(19) Europäisches Patentamt
European Patent Office
Office européen des brevets

(11) Veröffentlichungsnummer : 0 304 780 B1

(12) EUROPÄISCHE PATENTSCHRIFT

(45) Veröffentlichungstag der Patentschrift : 07.08.91 Patentblatt 91/32

(51) Int. Cl.[5] : A61K 49/02, C07F 13/00, A61K 43/00

(21) Anmeldenummer : 88113317.7

(22) Anmeldetag : 17.08.88

# (54) Verfahren zur Markierung von Substanzen mit Technetium oder Rhenium.

(30) Priorität : 27.08.87 DE 3728600

(43) Veröffentlichungstag der Anmeldung : 01.03.89 Patentblatt 89/09

(45) Bekanntmachung des Hinweises auf die Patenterteilung : 07.08.91 Patentblatt 91/32

(84) Benannte Vertragsstaaten : AT BE CH DE ES FR GB GR IT LI LU NL SE

(56) Entgegenhaltungen :
US-A- 4 431 627
CHEMICAL ABSTRACTS, Band 108, No. 4, 25.Januar 1988, Columbus, Ohio, US; J. FRANZ et al. "The production of technetium-99m-labeled conjugated antibodies using a cyclam-based bifunctional chelating agent." Seite 268, rechte Spalte, Zusammenfassung Nr. 2 685z. & Nucl. Med. Biol. 1987, 14(6), 569-72.
CHEMICAL ABSTRACTS, Band 109, Nr. 1, 04. Juli 1988, Columbus, Ohio, US; J. R. MORPHY et al. "Antibody labeling with functionalized cyclam macrocyles." Seite 298, linke Spalte, Zusammenfassung Nr. 3 066f & J. Chem. Soc., Chem. Commun. 1988, (3), 156-8.

(56) Entgegenhaltungen :
THE INTERNATIONAL JOURNAL OF APPLIED RADIATION AND ISOTOPES, Band 33, Nr. 10, Oktober 1982, W.A. VOLKERT et al. "Labeling of amine ligands with 90mTc in aqueous solutions by ligand exchange." Seiten 891-896.
RADIOCHEMICAL AND RADIOANALYTICAL LETTERS, Band 47, Nr. 1-2, 25.März 1981; J. SIMON et al. "Radiochemical characterization of Tc-cyclam." Seiten 111-124.

(73) Patentinhaber : HOECHST AKTIENGESELLSCHAFT
Postfach 80 03 20
W-6230 Frankfurt am Main 80 (DE)

(72) Erfinder : Bremer, Karl-Heinz, Dr.
Am Rehsteig 19
W-6232 Bad Soden am Taunus (DE)
Erfinder : Kuhlmann, Ludwig, Dr.
Berliner Strasse 57
W-6093 Flörsheim am Main (DE)
Erfinder : Magerstädt, Michael, Dr.
Schwedenstrasse 5
W-6237 Liederbach (DE)
Erfinder : Schwarz, Alexander, Dr.
Wiesenring 61
W-6093 Flörsheim am Main (DE)
Erfinder : Steinsträsser, Axel, Dr. Dr.
Zum Morgengraben 26
W-6237 Liederbach (DE)

EP 0 304 780 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Markierung von Substanzen mit — insbesondere radioaktiven — Technetium- bzw. Rheniumisotopen mit Hilfe von Komplexbildnern, sowie die Verwendung dieser markierten Substanzen.

Die Anwendungsmöglichkeiten von Radionukliden in der Technik sind sehr vielfältig. Sie erstrecken sich beispielsweise auf die Prüfung einer Durchmischung, auf die verdünnungsanalytische Bestimmung von Mengen oder Volumina, auf die Messung der Strömungsgeschwindigkeit und Aufnahme der Verweilzeit an kontinuierlich arbeitenden Produktionsanlagen.

Meist genügt jedoch nicht die bloße Beimischung eines radioaktiven Nuklids, sondern beispielsweise bei der "Verfolgung" einer bestimmten Komponente eines Systems, muß das radioaktive Nuklid physikalisch oder besser noch chemisch an mindestens eine Verbindung der zu untersuchenden Komponente gekoppelt werden und dies nach Möglichkeit ohne die physikalischen und chemischen Eigenschaften der betreffenden Verbindung zu beeinflussen.

In den vergangenen Jahren wuchs das Verlangen, chemische Verbindungen mit radioaktiven Nukliden zu markieren. Vor allem im Bereich der medizinischen Diagnostik, wo pathologische Zustände bereits durch Substanzen angezeigt werden können, die im Organismus nur in ppm oder gar in noch geringeren Konzentrationen vorkommen, kann man heute nicht mehr auf radioaktiv markierte Substanzen verzichten.

Insbesondere Technetium-99m ist wegen seiner günstigen physikalischen Eigenschaften (Fehlen einer Korpuskularstrahlung, $\gamma$-Energie von 140 keV und Halbwertszeit von 6 Stunden) und der damit verbundenen geringen Strahlenbelastung zum wichtigsten Radionuklid in der nuklearmedizinischen Diagnostik geworden.

Technetium-99m, das sich aus Nuklidgeneratoren gewinnen läßt, liegt zunächst als Pertechnetat vor, das in dieser Form, z.B. für die Schilddrüsen- und Hirnszintigraphie geeignet ist. Die Szintigraphie anderer Organe mittels Technetium-99m gelingt mit Hilfe bestimmter "Transportsubstanzen", die einerseits in der Lage sind Technetium zu binden, und andererseits das Radionuklid im Zielorgan mit hoher Selektivität anzureichern. Zur Markierung der organspezifischen "Transportsubstanz" mit Technetium-99m muß das aus dem Nuklidgenerator eluierte Pertechnetat zuerst in eine niedrigere Oxidationsstufe überführt werden. In dieser reduzierten Form bildet Technetium mit der organspezifischen Substanz mehr oder weniger stabile Verbindungen. Für die Knochenszintigraphie werden z.B. Tc-99m-Phosphorsäure-Derivate, vor allem organische Phosphonsäuren, eingesetzt. So liegt in der im Europäischen Patent 2485 beschriebenen Markierungseinheit das Natriumsalz der 3,3-Diphosphono-1,2-propandicarbonsäure als organspezifische "Transportsubstanz" vor. In dem Europäischen Patent 108.253 werden Tc-99m-Tri- und Tetraphosphonsäuren zur szintigraphischen Darstellung des RES, insbesondere der Leber, beschrieben. Der Tc-99m-Komplex mit Diethylentriaminpentaessigsäure (DTPA) findet zur Diagnostik von Nierenerkrankungen bzw. pathologischen Hirnprozessen Anwendung.

Zur Markierung bestimmter Substanzen mit Technetium-99m und zur Herstellung von für den klinischen Routinebedarf geeigneten Testkits wurden spezielle Verfahren entwickelt und beschrieben. Zur Präparierung von Markierungsbestecken für biologisch interessante Makromoleküle, insbesondere Porphyrine, Dextrane, Cytochrome und Myoglobin, wird eine Methode beschrieben (G.D. Zanelli, D. Ellison, M.P. Barrowcliffe, Nucl. Med. Commun. 8, 199-206, 1987), bei der die zu markierende Substanz zusammen mit p-Aminobenzoesäure und einer salzsauren $SnCl_2$-Lösung lyophilisiert wird. Zur Rekonstitution und Markierung dieses Kits gibt man Tc-99m-Generatoreluat, das vorher mit genügend Pufferlösung, z.B. Citrat-Natriumchlorid-Puffer pH 9,5, verdünnt wurde, hinzu. Für säureempfindliche Substanzen ist diese Methode jedoch nicht geeignet.

Bei einem anderen Verfahren (E.K.J. Pauwels, R.I.J. Feitsma, Patentanmeldung Int. Publication No. WO 86/03010) wird durch vierstündiges Erhitzen in stark salzsaurer Lösung auf 140°C Tc-99m-Pertechnetat zuerst reduziert und an einer Verbindung, die eine Aminogruppe enthält, z.B. Dimethylformamid, gebunden. Das reaktionsfähige Tc-99m-markierte Zwischenprodukt, das als schwerlösliche kristalline Substanz ausfällt, wird in einer Pufferlösung, z.B. Natriumcarbonatlösung, durch einstündige Inkubation bei Raumtemperatur mit der zu markierenden Verbindung umgesetzt. Die Methode arbeitet zwar zinnfrei, ist aber wegen der aufwendigen Verfahrensschritte für die Routineanwendung kaum geeignet.

Zur Markierung von Proteinen, insbesondere Antikörpern, kennt man zwei verschiedene Wege. Bei der direkten Methode wird das reduzierte Technetium-99m durch Donorgruppen (Amino-, Amid-, Thiol- etc.) des Proteins gebunden.

Solche Verfahren sind in dem Europäischen Patent 5638 und dem US-Patent 4.478.815 beschrieben. Dort werden Zinn-II-Salze im Überschuß zur gleichzeitigen reduktiven Spaltung von Disulfid-Brücken und zur Reduktion des zugesetzten Tc-99m-Pertechnetats benutzt. Im allgemeinen benötigt man zur Spaltung der —S-S-Bindung längere Inkubationszeiten (24 Stunden), wobei $F(ab')_2$-Fragmente partiell zu F(ab)-Fragmenten gespalten werden. Neuere Literaturangaben (z.B. Journal of Nuclear Medicine 27 (1986), Seite 685-93 und 1315-20 sowie International Journal of Nuclear Medicine Biology 12 (1985) Seiten 3-8) zeigen, daß das Verhältnis der beiden Fragmente abhängig ist von der "Bezinnungsreaktion" und daß sich das Verhältnis der beiden Komponenten nach der Tc-99m-Markierung nicht mehr nennenswert ändert, wobei die Hauptkomponente Tc-99m-markiertes F(ab') ist. In allen Fällen mußte das markierte F(ab')-Fragment nachgereinigt werden, da trotz mindestens 30-minütiger Reaktionszeit keine quantitative Umsetzung des Pertechnetats erreicht wurde.

Bei einer schnellen chemischen Methode zur Tc-99m-Markierung humaner Plasmaproteine (D.W. Wong, F. Mishkin, T. Lee, J. Nucl. Med. 20, 967-72, 1979) wird Pertechnetat zuerst in saurer Lösung durch Zinn-II-Ionen reduziert und das reduzierte Technetium dann mit dem Protein umgesetzt.

Unter Zuhilfenahme von bifunktionalen Komplexbildnern läßt sich eine stabile Markierung von Substanzen mit Radioisotopen erreichen.

In dem US-Patent 4.479.930 werden die cyclischen Anhydride von DTPA und EDTA als Chelatisierungsmittel nicht nur für In-111 und Ga-67, sondern auch für Tc-99m angeführt. In dem europäischen Patent 35765 wird die Verwendung von Deferoxamin als Komplexierungsagens für Technetium-99m an Proteine erwähnt. In der internationalen Patentanmeldung WO 85/3063 werden die partiell reduzierten Disulfid-Brücken im Antikörper mit dem Natriumsalz des Tetrachlornitridotechnetats, das durch Reaktion von Pertechnetat mit Natriumazid vorher präpariert werden muß, umgesetzt. In der europäischen Patentanmeldung 194853 benutzt man ebenfalls durch Reduktion in Antikörperfragmenten erzeugte freie Thiolgruppen zur Bindung von [(7-Maleimidoheptyl)imino-bis(ethylennitrilo)]-tetraessigsäure als Chelatkomplex. Die Kupplung des Komplexes an den Antikörper erfolgt über die Reaktion der SH-Gruppen mit der Doppelbindung im Maleinimidteil der Komplexverbindung, während das radioaktive Metallion über die Nitrilodiessigsäure-Reste komplexiert wird.

Metallothionein, ein metallbindendes Protein mit einem Molekulargewicht von 6000 und einem hohen Cysteinanteil im Molekül wurde als Komplexbildner in Antikörper eingeführt (G.L. Tolman, R.J. Hadjian, M.M. Morelock et al., J. Nucl. Med. 25, 20, 1984). Durch Austausch mit Tc-99m-Glucoheptonat ließ sich das Antikörper-Metallothionein-Konjugat mit Technetium markieren. Der Austausch blieb jedoch unvollständig, so daß eine Nachreinigung erforderlich war. Mehrere Bisthiosemicarbazon-Liganden wurden ebenfalls als bifunktionelle Chelatbildner beschrieben (Y. Arano, A. Yokoyama, H. Magat et al., Int. J. Nucl. Med. Biol. 12, 425-30, 1986). p-Carboxyethylphenylglyoxal-di(N-methylthiosemicarbazon) wurde mit humanem Serumalbumin konjugiert. Der Tc-99m-markierte 1 : 1-Komplex zeigte eine gewisse Instabilität, während Komplexe mit einem höheren Verhältnis als 1 : 1 eine vermehrte Leberspeicherung aufwiesen. Die Kopplung eines Diamid-dimercaptid-$N_2S_2$-Liganden an Proteine (A.R. Fritzberg, S. Kasina, J.M. Reno et al., J. Nucl. Med. 27, 957-958, 1986) erfolgt über eine zusätzliche funktionelle Gruppe. So wurde z.B. 4,5-Di(S-ethylcarbonylmercaptoacetamid)-pentanoyl-N-hydroxysuccinimid mit einem anti-Melanomantikpörper umgesetzt. Das entstandene Konjugat wurde bei pH 8 und 50°C mit Tc-99m-Tartratlösung inkubiert. Nach einer Stunde waren 78% des Technetiums vom Tartrat auf den Antikörper übertragen.

Um Technetium-99m diagnostisch breit nutzen zu können, ist es notwending, dieses Nuklid in das zu untersuchende Organ selektiv zu transportieren. Aus anderen Organen oder Organsystemen sollte das Technetium-99m wieder schnell eliminiert oder überhaupt nicht hingebracht werden, um jede unnötige Strahlenbelastung für den Patienten zu vermeiden. Zu diesem Zweck werden bisher vorwiegend Substanzen eingesetzt, die direkt mit Technetium-99m markierbar sind und eine hohe Organspezifität aufweisen. Darüber hinaus gibt es jedoch eine Reihe von Substanzen, die zwar eine hohe Organspezifität aufweisen, aber nicht direkt markierbar sind. Dies können Proteine (Fibrinogen, Humanserumalbumin), Enzyme (Streptokinase, Lactatdehydrodgenase), Zucker (Dextran, Glucose) oder auch Polymere sein. Dazu gehören ebenfalls niedermolekulare Substanzen wie etwa Fettsäuren, die durch den hohen Energiebedarf des Herzens sich im Myocardgewebe anreichern. Um diese Substanzen markieren zu können, werden sie mit Komplexbildnern gekoppelt, die ihrerseits Technetium-99m fest binden können.

Als geeignete Komplexbildner für die Komplexierung von Technetium- und Rheniumisotopen sind makrozyklische Amine, u.a. auch Cyclame, bekannt. Die Komplexierungsausbeute liegt für den Technetium-Cyclam Komplex bei 99% unter geeigneten Bedingungen. Einzelheiten über Technetium-Aminkomplexe sind in D.E. Troutner, J. Simon, A.R. Ketring, W.A. Volkert, R.A. Holmes, J. Nucl. Med. 21 (1980), 443 oder S.A. Zuckman, G.M. Freeman, D.E. Troutner, W.A. Volkert, R.A. Holmes, D.G. van der Keer, E.K. Barefiled, Inorg. Ch. 20 (1981), 3386 oder J. Simon, D. Troutner, W.A. Volkert, R.A. Holmes, Radiochem. Radioanal. Lett. 47 (1981), 111 erwähnt. Auch substituierte Cyclame sind sowohl am 1-Stickstoff, als auch am 6-Kohlenstoff substituiert, bekannt (A.R. Ketring, D.E. Troutner et al., Int. J. Nucl. Med. Biol. 11 (1984), 113 oder J. Simon. Diss. Abstr. Int. B42 (1981), 645 oder M. Struden, T.A. Kaden, Helv. Chim. Acta 69 (1986), 2081 oder E. Kimura, R. Machida, M. Kodama, J. Am. Chem. Soc. 106 (1984), 5497.

Alle bisherigen Versuche, Amin- und auch andere Liganden an Proteine zu konjugieren (s. Fritzberg et al., J. Nucl. Med. 27 (1986), 957 oder Tolman et al., J. Nucl. Med. 25 (1984), 20 oder Arano et al., Int. J. Nucl. Med. Biol. 12 (1986) 425) führten zu Produkten, die die hohen in vivo Stabilitätsansprüche nicht oder nur teilweise erfüllten.

Es wurde nun ein Verfahren gefunden, daß es erlaubt, Substanzen mit Hilfe substituierter makrozyklischer Amine, insbesondere Cyclame, mit Technetium bzw. Rheniumisotopen zu markieren.

Unter den "Substanzen" die mit Hilfe des erfindungsgemäßen Verfahrens markierbar sind, werden in erster Linie solche Verbindungen verstanden, die in der medizinischen Diagnostik als "Transportsubstanzen" eingesetzt werden können, also meist Verbindungen, die eine hohe Organspezifität aufweisen wie Antikörper, Antikörperfragmente wie $F(ab')_2$- oder F(ab')-Fragmente, Proteine wie Fibrinogen, Humanserumalbumin, Steroide, Lipide, Enzyme wie Streptokinase, Lactatdehydrogenase, Zucker wie Dextran, Glucose oder auch Polymere. Andererseits können aber auch generell solche Substanzen mit Hilfe des erfindungsgemäßen Verfahrens markiert werden, die mit der funktionellen Gruppe an der Seitenkette des makrocyclischen Amins unter Ausbildung einer chemischen Bindung reagieren. Gedacht ist hierbei an die "Überwachung" von chemischen Substanzen in Produktionsanlagen, die Bestimmung ihrer Konzentration, Strömungsgeschwindigkeit, Verweilzeit etc.

Die Erfindung betrifft somit ein Verfahren zur Markierung von Substanzen mit Technetium- bzw. Rheniumisotopen, das dadurch gekennzeichnet ist, daß

a) die zu markierende Substanz mit einem N-substituierten oder C-substituierten makrocyclischen Aminderivat der Formel I und/oder II

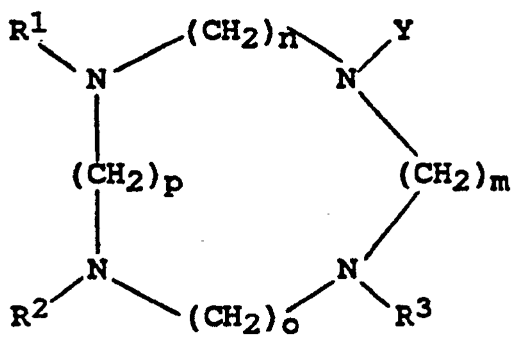

I

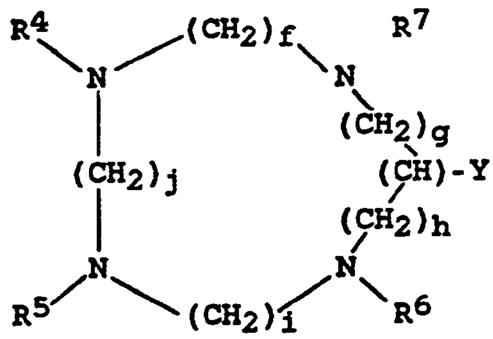

II

worin

$R^1$, $R^2$, $R^3$, $R^4$, $R^5$, $R^6$ und $R^7$ Wasserstoff, bedeuten und

m, n, o, p, f, h, i, j gleich oder verschieden sind und 1, 2, 3 oder 4 und g 0, 1, 2, 3 oder 4 bedeuten und

Y eine Gruppe der Formel $-X-NH_2$, $-X-NCS$, $-X-COOH$, $-X-OH$, $-X-N_2^+$ oder $-X-COCl$ oder eine Gruppe der Formel $-X-Z$ bedeutet, wobei Z, Fluor, Chlor, Brom oder Jod bedeutet und X eine Alkylengruppe mit 1 bis 40 C-Atomen bedeutet oder X eine ortho-, meta- oder para-Phenylen oder eine ortho-, meta- oder para-Phenylmethylgruppe bedeutet,

umgesetzt wird und die so erhaltene Verbindung mit Technetium-99m oder Rhenium-186 oder -188 markiert wird, in dem man sie mit Pertechnetat-99m oder Perrhenat-186 oder -188 und einem Reduktionsmittel für Pertechnetat-99m oder Perrhenat-186 oder -188 versetzt

oder daß man

b) zunächst den Technetium-99m- und/oder Rhenium-186 oder -188-Komplex durch Umsetzung einer Verbindung der Formel I und/oder II mit Pertechnetat-99m und/oder Perrhenat-186 oder -188 und einem Reduktionsmittel für Pertechnetat-99m und/oder Perrhenat-186 oder -188 herstellt und anschließend diesen Technetium-99m und/oder Rhenium-186 oder -188-Komplex mit der zu markierenden Substanz umsetzt.

Insbesondere betrifft die Erfindung ein Verfharen zur Markierung von Substanzen mit Technetium- bzw. Rheniumsiotopen, bei dem makrocyclische Amine der Formel I und/oder II verwendet werden, worin

$R^1$, $R^2$, $R^3$, $R^4$, $R^5$, $R^6$ und $R^7$ Wasserstoff bedeuten und

m, n, o, p, f, i und j gleich oder verschieden sind und

2 oder 3 und g 0, 1 oder 2 und h 1 oder 2 bedeutet und

Y eine Gruppe der Formel $-X-NH_2$, $-X-NCS$, $-X-COOH$, $-X-OH$, $-X-N_2^+$ oder $-X-COCl$ oder eine Gruppe der Formel $-X-Z$ bedeutet, wobei Z Fluor, Chlor, Brom oder Jod bedeutet und X eine Alkylengruppe mit 1 bis 20 C-Atomen bedeutet.

Ganz besonders bevorzugt ist ein Verfahren zur Markierung von Substanzen mit Technetium- bzw. Rheniumsiotopen, bei dem makrocyclische Amine der Formel I und/oder II verwendet werden, worin

$R^1$, $R^2$, $R^3$, $R^4$, $R^5$, $R^6$ und $R^7$ Wasserstoff bedeuten und

m, p und j 3 und n, o, f und i 2 und g und h 1 bedeuten und

Y eine Gruppe der Formel —X-$NH_2$ bedeutet, wobei X eine Alkylengruppe mit 1 bis 15 C-Atomen bedeutet.

Weiterhin betrifft die Erfindung die Verwendung der markierten Substanzen, insbesondere in der medizinischen Diagnostik.

Unter Aryl werden Phenyl und Naphthyl, insbesondere Phenyl verstanden. Alkylgruppen mit mehr als 2 C-Atomen können sowohl geradkettig als auch verzweigt sein. Bei der Alkylengruppe X handelt es sich bevorzugt um geradkettige Alkylene mit bis zu 40 C-Atomen, bevorzugt bis zu 20, besonders bevorzugt bis zu 15 C-Atomen.

Bei dem erfindungsgemäßen Verfahren wird entweder ein N-substituiertes oder C-substituiertes makrocyclisches Aminderivat, das am Ende des Substituenten eine funktionelle Gruppe trägt, mit Hilfe dieser funktionellen Gruppe an die zu markierende Substanz gebunden. Gegebenenfalls nach entsprechender Reinigung des Konjugats (z.B. durch Ultrafiltration oder Dialyse bei Proteinen oder Polymeren, durch Säulenchromatographie beiniedermolekularen Substanzen wie Steroiden oder Lipiden) werden Technetium-99m in der Form von Pertechnetat bzw. Rhenium-186 oder -188 in der Form von Perrhenat und ein geeignetes Reduktionsmittel zur Reduktion des Pertechnetats bzw. Perrhenats in die für die Komplexierung benötigte Oxidationsstufe, in beliebiger Reihenfolge oder gemeinsam zugegeben. Das markierte Substrat wird gegebenenfalls nochmals gereinigt.

Alternativ kann auch zunächst der Technetium- bzw. Rheniumkomplex des cyclischen Amins erzeugt werden und dann dieser mit der Substanz zum Konjugat umgesetzt werden. Hierbei verläuft die Komplexierung und Reduktion wie oben beschrieben. Vorzugsweise wird die Komplexierungsreaktion bei basischem pH (7 bis 14) durchgeführt. Vorzugsweise werden makrocyclische Amine der Formel III oder IV

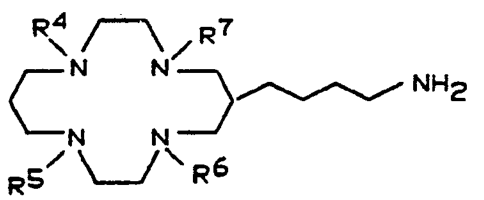

oder

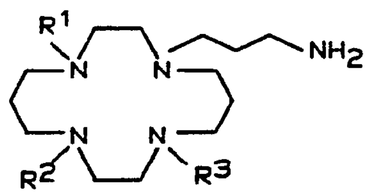

III IV

als Komplexbildner eingesetzt.

Die Herstellung der makrocyclischen Amine gemäß Formel I oder II gelingt am einfachsten durch Umsetzungen von Bis-(ω-aminoalkyl)-aminen, welche zunächst tritosyliert und anschließend in das Dinatriumsalz überführt werden, mit dreifach tosyliertem α,ω-Dihydroxydialkylamin, gefolgt von einer Detosylierung. Eine entsprechende Synthesemethode ist von J. Richman und T. Atkins in J. Am. Chem. Soc. 96 (1974), 2268 beschrieben. Die Reste $R^1$ bis $R^7$ können nach literaturbekannten Verfahren, z.B. durch Alkylierung mit Alkylhalogeniden eingeführt werden.

Die Ankoppelung der exocyclischen Seitenkette mit endständiger reaktiver Gruppe an eine NH-Gruppe geschieht beispielsweise durch Umsetzung der makrocyclischen Amine mit ω-Nitroalkylcarbonsäurehalogeniden, bevorzugt -chloriden und anschließender Hydrierung, wobei die Carbonyl- und die Nitrogruppe zu einer $CH_2$- bzw. $NH_2$-Gruppe reduziert werden. Eine analoge Umsetzung mit Bromiden ist beispielsweise beschrieben in A.R. Ketring, D.E. Troutner et al., Int. J. Nucl. Med. Biol., 11 (1984), 113.

Zur Herstellung der C-substituierten makrocyclischen Amine kann man beispielsweise folgendermaßen vorgehen : N-N'-(Aminoalkyl)-α,ω-alkandiamin wird mit Tosylchlorid und Natriumalkoholat in das Tetratosyldinatriumderivat überführt, welches mit einem (vorzugsweise) Tosylaminoalkyl-α,ω-alkyltosylat zu dem C-Aminoalkyl substituierten Tetraazacycloalkan umgesetzt wird.

Alle diese Varianten der Synthese von makrocyclischen Aminen beruhren mehr oder weniger auf dem Verfahren von J. Richman und T. Atkins (s. loc. cit). Durch einfache Abwandlungen dieses Verfahrens lassen sich die unterschiedlichsten makrocyclischen Amine — z.B. solche mit unterschiedlich langen Alkylenketten oder auch solche mit C-substituierten, exocylischen Seitenketten — herstellen.

Die meisten der oben beschriebenen Ausgangsverbindungen, z.B. viele N,N'-Diaminoalkyl-α,ω-alkandiamine und viele "Y"-substituierte α,ω-Alkandiole, sind käuflich oder können auf einfache Weise hergestellt werden.

Die Reduktion des Pertechnetats bzw. des Perrhenats kann nach literaturbekannten Verfahren erfolgen, bevorzugt mit einer Zinn-II-Verbindung. Besonders bevorzugt erfolgt die Reduktion mit einem komplexstabilisierten Zinn-II-Salz, nach einem "Markierungsverfahren", wie dies in der deutschen Offenlegungsschrift DE-A 3728599 vorgeschlagen wurde. Dabei wird zunächst die Zinn-II-Verbindung mit einem Komplexbildner, vorzugsweise einer Phosphorverbindung wie einem Phosphonat oder Pyrophosphat versetzt, der dafür sorgt, daß die Zinnverbindung vor allem im physiologischen pH-Bereich in Lösung bleibt. Diese komplexstabilisierte Zinn-II-Salz-Lösung kann dann entweder zu der zu markierenden Substanz gegeben werden, worauf die Zugabe der Pertechnetat- bzw. Perrhenat-Lösung erfolgt oder aber zu einer Mischung aus der zu markierenden Substanz und der Pertechnetat- bzw. Perrhenat-Lösung.

Im folgenden wird die Erfindung anhand von Beispielen näher erläutert und in den Patentansprüchen definiert.

**Beispiel 1**

Darstellung des N-substituierten Cyclams 1-(3-Aminopropyl)-1,4,8,11-tetraazacyclotetradecan

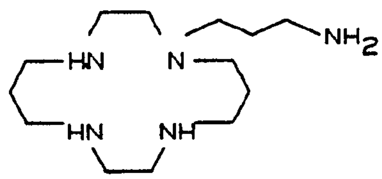

a) 5 g 3-Nitropropionsäure werden in ca. 40 ml $SOCl_2$ gelöst und 2 h unter Rückfluß gekocht. Das $SOCl_2$ wird abgezogen und das Produkt vakuumgetrocknet.

b) 6,6 g Cyclam (1,4,8,11-Tetraazacyclotetradecan) und 0,18 g $Na_2CO_3$ werden in 100 ml $CHCl_3$ bei 0°C vorgelegt. Dazu werden 0,45 g 3-Nitropropionsäurechlorid getropft. Nach Rühren über 24 h wird 8 h unter Rückfluß gekocht, dann wird die Lösung eingeengt und getrocknet. Der Rückstand wird in wäßriger HCl aufgenommen (pH 1,5) und 2 mal mit je 100 ml Diethylether extrahiert. Wieder wird zur Trockne eingeengt. Der Rückstand wird in 100 ml 40% NaOH gegeben, tituriert und die Mischung 2 mal mit je 50 ml $(C_2H_5)_2O$ extrahiert. Die Etherphase wird verworfen. Extraktion der Base mit 2 mal 100 ml Ethylacetat ergibt nach Abtrennen und Trocknen der organischen Phase einen gelblichen Rückstand, von dem bei 100°C/0,1 Torr überschüssiges Cyclam absublimiert wird. Nach Umkristallisation aus $H_2O$ erhält man das (3-Nitropropyl)-amidocyclam als hygroskopischen gelblichen Feststoff (Zersetzung bei 175°C), charakterisiert durch C, H, N-Analyse, Massenspektrum und $^1$H-NMR sowie IR.
Ausbeute : 0,22 g (25%)

Zu einer wäßrigen $H_2$-gesättigten Lösung eines Katalysators aus 10% Pt auf Aktivkohle wird eine wäßrige Lösung von 0,2 g (3-Nitropropyl)-amidocyclam und Ammoniak (pH 10) bei 0°C gegeben. Das Gemisch wird unter $H_2$-Atmosphäre gebracht. Die Reaktion ist beendet, wenn kein $H_2$ mehr verbraucht wird. Die Lösung wird über Kieselgur filtriert und gefriergetrocknet. Das 1-(3-Aminopropyl)-1,4,8,11-Tetraazacycloetetradecan wird als gelblicher Feststoff gewonnen und durch C, H, N-Analysen, Massenspektren und $^1$H-NMR charakterisiert.
Aubseute : 100 mg ($\hat{=}$ 58% d. Th.)

**Beispiel 2**

Darstellung von 6-(4-Aminobutyl)-1,4,8,11-Tetraazacyclotetradecan

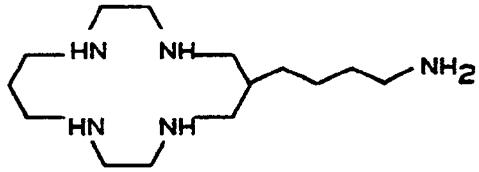

**a) Synthese von TsHN-$(CH_2)_2$-NTs-$(CH_2)_3$-NTs-$(CH_2)_2$-NHTs**

2 g N,N'-(Diaminoethyl)-1,3-propandiamin werden zusammen mit 2 g NaOH in 250 ml Wasser gelöst und

während 30 Minuten werden 9,4 g p-Toluolsulfonylchlorid, gelöst in 80 ml Diethylether, unter starkem Rühren bei Raumtemperatur zugetropft. Nach 1 h wird durch Einleiten von $N_2$ der Ether langsam verdampft, wobei das tetratosylierte Produkt anfällt. Es wird von der Lösung abfiltriert, sorgfältig mit viel Wasser gewaschen und vakuumgetrocknet. Das farblose, feste Produkt kann an seinem $^1$H-NMR-Spektrum in $CDCl_3$ identifiziert werden. Ausbeute 6,5 g (67% d. Th.)

**b) Synthese von NaTsN-$(CH_2)_2$-NTs-$(CH_2)_3$-NTs-$(CH_2)_2$-NTsNa**

Das Tetratosylat (6,5 g) wird in ca. 200 ml $C_2H_5OH$ vorgelegt und eine Lösung von 0,4 g Na in 40 ml $C_2H_5OH$ dazugetropft. Nach 1 h Rückflußkochen wird die Lösung am Rotationsverdampfer eingeengt und das Natriumsalz des Tetratosylats vakuumgetrocknet. Ausbeute 6,8 g, Sublimation/Zersetzung bei 110°C.

**c) Synthese von 2-(3-Cyanopropyl)-malonsäurediethylester**

5,2 g NaH werden 3 mal mit Petrolether gewaschen, dann in 300 ml THF aufgenommen. Bei 0°C wird Diethylmalonat (16 g) unter Stickstoff langsam zugetropft, dann das Gemisch auf Raumtemperatur erwärmt, 2 h rückflußgekocht, dann wieder auf 0°C gekühlt, gefolgt von Zutropfen des 4-Brombutyronitrils (8,2 ml) in ca. 20 ml Tetrahydrofuran. Nach 15-stündigem Rühren bei Raumtemperatur wird für 1 h rückflußgekocht, abgekühlt, vom NaBr abfiltriert und zu einem Öl eingeengt. Nach Zugabe von ca. 50 ml $CH_2Cl_2$ wird 2 mal mit je 100 ml $H_2O$ extrahiert und dann die organische Phase abgetrennt, über $MgSO_4$ getrocknet und eingeengt. Der ölige Rückstand wird im Vakuum destilliert und geht bei 105-112°C/0,1-0,5 mm Hg über. $^1$H-NMR in $CDCl_3$ zeigt das Produkt. Ausbeute 6,02 g (32% d. Th.)

**d) Synthese von 2-(Aminobutyl)-propan-1,3-diol**

6 g 2-(Butylnitril)-malonsäurediethylester werden bei −10°C zu einer Suspension von 3 g $LiAlH_4$ in 100 ml trockenem Diethylether unter $N_2$ getropft. Das Gemisch wird langsam auf Raumtemperatur erwärmt und dann 3 h rückflußgekocht. Nach erneutem Abkühlen auf −10°C wird das überschüssige Hydrid mit Wasser zerstört. Der Niederschlag wird über eine Fritte abgetrennt und mehrmals mit Diethylether gewaschen. Die Etherlösung wird über $MgSO_4$ getrocknet, filtriert und am Rotationsverdmapfer eingeengt. Ausbeute 0,66 g des 2-(Aminobutyl)-propan-1,3-diol im $^1$H-NMR und durch C, H, Analyse nachweisbar. Glasartiger Feststoff, Fp. : 126°C (ab 60°C honigartig).

**e) Überführung des 2-(Aminobutyl)-propan-1,3-diols in das O,O,N-Tritosylat**

Das 2-(Aminbutyl)-propan-1,3-diol (0,6 g) wird in 50 ml $CH_2Cl_2$ vorgelegt, dann gibt man 1,22 ml Pyridin dazu und tropft zu dieser Lösung bei 20°C über 30 Minuten 2,35 g TsCl in 20 ml $CH_2Cl_2$. Nach 20-stündigem Rühren wird mehrmals mit 2 N-HCl gewaschen, die organische Phase abgetrennt, über $MgSO_4$ getrocknet und am Rotationsverdampfer eingeengt. Das Produkt — tosyliertes 2-(Aminobutyl)-propan-1,3-diol — kann durch Säulenchromatographie mit Butanol/Kieselgel gereinigt werden. Nachweis durch C, H, N Analyse und $^1$H-NMR. Ausbeute : 2,36 g (95%).

**f) Cyclisierung**

1,74 g des Dinatriumsalzes aus Beispiel 2b) werden in 5 ml Dimethylformamid vorgelegt, auf 100°C erhitzt und das Dioltritosylat aus Beispiel 2e), gelöst in 10 ml DMF, bei 100°C dazugetropft. Nach 4 h Rühren bei 100°C wird die Lösung auf Rautemperatur gekühlt und dann noch weitere 15 h gerührt. Nach Zugabe von ca. 10-15 ml $H_2O$ fällt tosyliertes 6-(4-Aminobutyl)-1,4,8,11-tetraazacyclotetradecan aus, das mehrmals mit Wasser gewaschen und getrocknet wird. Ausbeute 1,43 g (66% d. Th.). Charakterisierung durch $^1$HNMR in $CDCl_3$. Zersetzung ab 132°C, vollständig zersetzt bei 236°C.

**g) Detosylierung**

Das Pentatosylat aus Beispiel 2f) (1,4 g) wird in 20 ml konz., $H_2SO_4$ gegeben und bei 95°C gemischt. Sobald eine Probe wasserlöslich ist, wird auf −10°C abgekühlt und das Produkt mit 20 ml Diethylether ausgefällt. Nach Filtration wird der graue, getrocknete Feststoff in 40% NaOH (40 ml) gegeben und dann mehrmals mit Diethylether extrahiert. Aus dem Ether erhält man das 6-(4-Aminobutyl)-1,4,8,11-tetraazacyclotetradecan, welches chromatographisch gereinigt werden kann. Ausbeute : 0,3 g (85%). Charakterisierung durch C, H, N-Analyse, Massenspektrum und $^1$H-NMR.

**Beispiel 3**

Synthese von 6-(11-Hydroxyundecyl)-1,4,8,11-Tetraazacyclotetradecan

a) Synthese des 11-Bromundecansäuremethylesters

In einen Dreihalskolben mit Kühler, Blasenzähler, Thermometer und Tropftrichter werden 26,5 g (0,10 mol) 11-Bromundecansäure vorgelegt und 17,8 g (0,15 mol) Thionylchlorid unter Eiskühlung zugetropft, daß die Temperatur 20°C nicht übersteigt. Anschließend wird im Wasserbad auf 70°C erwärmt und bis zur Beendigung der Gasentwicklung (HCl, $SO_2$) gerührt. Das unverbrauchte Thionylchlorid wird im Wasserstrahlvakuum in eine Kühlfalle kondensiert.

Zu dem ungereinigten 11-Bromundecansäurechlorid werden 3,52 g (0,11 mol) wasserfreies Methanol gegeben und bis zur Beendigung der Salzsäureentwicklung im Wasserbad bei 40°C gerührt. Das erhaltene Rohprodukt wird über eine Vigreux-Kolonne destilliert (Sdp. : 105 bis 108°C/0,1 mbar).
Ausbeute : 26,19 g (93,8%) Charakterisierung durch $^1$H-NMR, Massenspektrum, C,H-Analyse

b) Synthese des 12,12-Dicyanododecansäuremethylesters

In einem Dreihalskolben mit Rührer, Rückflußkühler und Tropftrichter werden 100 ml wasserfreies Tetrahydrofuran vorgelegt und 11,22 g (0,10 mol) Kalium-tert.-butylat unter Rühren suspendiert. Dazu werden 6,61 g (0,10 mol) 1,1-Dicyanomethan, gelöst in 20 ml wasserfreiem Tetrahydrofuran, unter Eiskühlung zugetropft, daß die Temperatur nicht über 10°C steigt. Anschließend gibt man 28,0 g (0,10 mol) 11-Bromundecansäuremethylester zu und kocht 15 h unter Rückfluß. Nach Abkühlung wird das ausgefallene Kaliumbromid abfiltriert, das Filtrat am Rotationsverdampfer bis zur Trockene eingeengt, der Rückstand in 100 ml Diethylether aufgenommen und dreimal mit jeweils 50 ml Wasser gewaschen. Die etherische Phase trocknet man über Natriumsulfat engt am Rotationsverdampfer bis zur Trockene ein und destilliert das erhaltene Rohprodukt über eine kurze Destillationsbrücke (Sdp. : 150 bis 160°C/0,6 mbar).
Ausbeute : 25,56 g (96%)
Schmp. : 36°C, Charakterisierung durch $^1$H-NMR, Massenspektren, C,H,N-Analyse

c) Reduktion von 12,12-Dicyanododecansäuremethylester zu 12-Aminomethyl-13-aminotridecanoldihydrochlorid

In einem Zweihalskolben mit Tropftrichter, Rückflußkühler und aufgesetztem Blasenzähler werden 5 g 12,12-Dicyanododecansäuremethylester (19 mmol) in 200 ml Tetrahydrofuran gelöst und auf 0°C gekühlt. Dazu werden 200 ml 1 molare $BH_3$-THF-Komplexlösung in Tetrahydrofuran getropft. Dann wird 3 Stunden lang am Rückfluß gekocht, auf Raumtemperatur gekühlt, 1 bis 2 Stunden gerührt, wieder auf 0°C gekühlt und vorsichtig trockenes Methanol (ca. 100 bis 150 ml) zugetropft, um überschüssiges Boran zu zerstören. Die Lösungsmittel werden abrotiert, dann wird der klebrige Rückstand in ca. 100 ml Methanol aufgenommen. Wieder wird das Methanol abrotiert und nochmals der Rückstand in 100 ml Metahnol aufgenommen und einrotiert. Zu dem Rückstand gibt man trockenes Ethanol, bis gerade eine klare Lösung entsteht. Diese wird auf −10°C gekühlt und eine Stunde lang mit HCl-Gas behandelt. Das Gemisch wird dann 5 h lang rückflußgekocht. Nochmals kühlt man auf −10°C und sättigt die Lösung mit gasförmiger HCl. Nach Kühlen auf −18°C im festverschlossenen Kolben über Nach fällt das Produkt als weißer, kristalliner Niederschlag aus. Mehr rohes Produkt kann durch Einrotieren der Lösung erhalten werden. Das Rohprodukt wird durch Waschen mit wenig kaltem Ethanol und anschließendes Vakuumtrocknen gereinigt.
Ausbeute : 5,1 g (79%)
Charakterisierung durch $^1$H-NMR, IR und C,H,N-Analyse.

d) Synthese von N,N′-Bis-(p-toluolsulfonyl)-12-aminomethyl-13-aminotridecanol

0,9 mmol der Substanz aus Beispiel 3c werden in ca. 5 ml Pyridin gelöst und das Tosylchlorid wird unter Rühren bei 20°C fest zugegeben. Nach 20 h Rühren bei 20°C wird das Lösungsmittel evaporiert und der Rückstand in 2 N HCl aufgenommen. Die saure Lösung wird 2 mal mit Diethylether extrahiert, die Etherphase ergibt 250 mg leicht gelblichen Feststoff (50%). Charakterisierung durch $^1$H-NMR.

e) Synthese von N,N′-dinatrium-N,N′-bis(p-toluolsulfonyl)-12-aminomethyl-13-aminotridecanol

14 mg Na werden in 5 ml Ethanol gelöst und zu einer Lösung von 200 mg der Substanz aus Beispiel 3d in 10 ml Ethanol gegeben. Nach zweistündigem Rühren bei Raumtemperatur wird 2 h rückflußgekocht. Die Lösung wird einrotiert und der Rückstand vakuumgetrocknet. Charakterisierung des Produkts (226 mg, 97% d. Th.) durch $^1$H-NMR (farbloser Feststoff).

f) Darstellung von N,N′-Bis-(p-toluolsulfonyl)-N,N′-bis-[2-(toluolsulfonyloxy)-ethyl]-propan-1,3-diamin aus Bis-N,N′-(hydroxyethyl)-propandiamin-(1,3)

4 g N,N′-bis(2-hydroxyethyl)-propandiamin-(1,3) werden in 100 ml $CH_2Cl_2$ gelöst und bei 20°C mit 7,8 g Pyridin versetzt. Dann gibt man 18,8 g Tosylchlorid in 70 ml $CH_2Cl_2$ dazu. Nach 50 h bei 20°C wird die Lösung mit 2 N HCl (100 ml) versetzt und 3 mal mit je 50 ml $CH_2Cl_2$ extrahiert. Aus der über $Na_2SO_4$ getockneten, filtrierten und evaporierten organischen Phase wird der feste Rückstand wieder in 50 ml 2 N HCl aufgenommen, 3 mal mit je 50 ml Diethylether, dann 2 mal mit je 50 ml Ethylacetat extrahiert. Die Ethylacetatfraktion ergibt nach Trocknen über $Na_2SO_4$, Filtrieren und Einrotieren 5,7 g (30% d. Th.) eines gelblichen Feststoffs, der durch $^1$H-NMR und Dünnschichtchromatographie charakterisiert wurde.

g) Cyclisierung von N,N′-dinatrium-N,N′-bis(p-toluolsulfonyl)-12-aminomethyl-13-aminotridecanol mit N,N′-Bis-(p-toluolsulfonyl)-N,N′-bis-[2-(p-toluolsulfonyloxy)-ethyl]propan-1,3-diamin zu 6-(11-Hydroxyundecyl)-1,4,8,11-tetra-(p-toluolsulfonyl)-1,4,8,11-tetraazacyclotetradecan

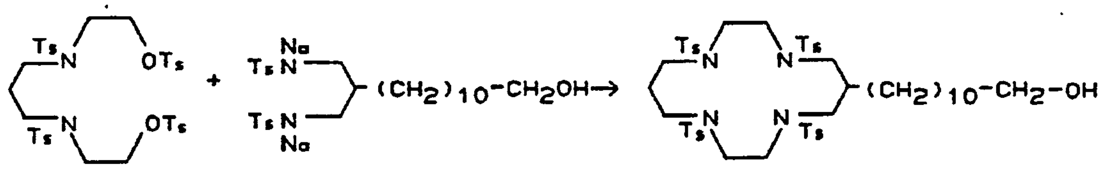

Bei 100°C wird eine Lösung von 220 mg der Substanz aus Beispiel 3e in 10 ml Dimethylformamid zu einer Lösung von 288 mg der Substanz aus Beispiel 3f in 10 ml Dimethylformamid getropft. Nach 4 h Rühren bei 100°C wird auf Raumtemperatur gekühlt und mit Wasser das Rohprodukt ausgefällt.

Der gelbe, klebrige Feststoff wird mit Wasser gewaschen (mehrmals), dann aus Aceton/Wasser umgefällt. Der getrocknete Feststoff (140 mg, 38%) kann durch Säulenchromatographie auf Kieselgel 60 mit Ether als Laufmittel weiter gereinigt werden (Rf = 0,72).

h) Detosylierung von 6-(11-Hydroxyundecyl)-1,4,8,11-tetra-(p-toluolsulfonyl)-1,4,8,11-tetraazacyclotetradecan mit $H_2SO_4$ zu 6-(11-Hydroxyundecyl)-1,4,8,11-tetraazacylotetradecan

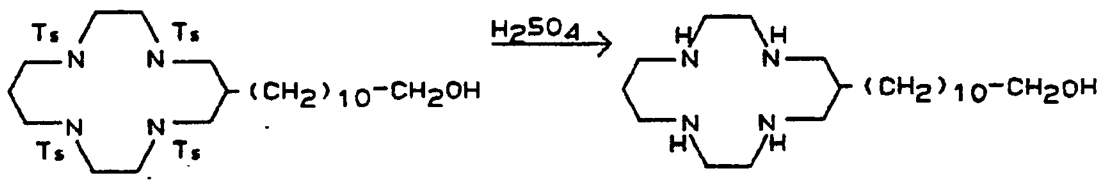


140 mg der Substanz aus Beispiel 3 g werden in ca. 5 ml konz. $H_2SO_4$ suspendiert und bei 100°C mehrere Stunden gerührt. Die Reaktion wird beendet, wenn eine Probe der Lösung wasserlöslich ist. Dann wird auf 4°C gekühlt und das Polysulfatsalz durch Zugabe von Diethylether gefällt und unter $N_2$ abfiltriert. Das Produkt wird in 40% NaOH gegeben und dann 6-(11-Hydroxyundecyl)-1,4,8,11-tetraazacyclotetradecan mit Benzol extrahiert. Das Produkt wird durch $^1$H-NMR, Massenspektrum, C,H,N-Analyse und IR-Spektrum charakterisiert, nachdem das Lösungsmittel abgezogen und der Rückstand vakuumgetrocknet wurde.
Ausbeute : 45 mg (87%).

**Beispiel 4**

Markierung eines COOH-Gruppen enthaltenden Polymers mit Technetium-99m mit Hilfe des bifunktionellen Cyclams aus Beispiel 1

a) Reaktion von Monomethoxypolyethylenglycolmonocarboxylat mit 1-(3-Aminopropyl)-1,4,8,11-tetraazacyclotetradecan

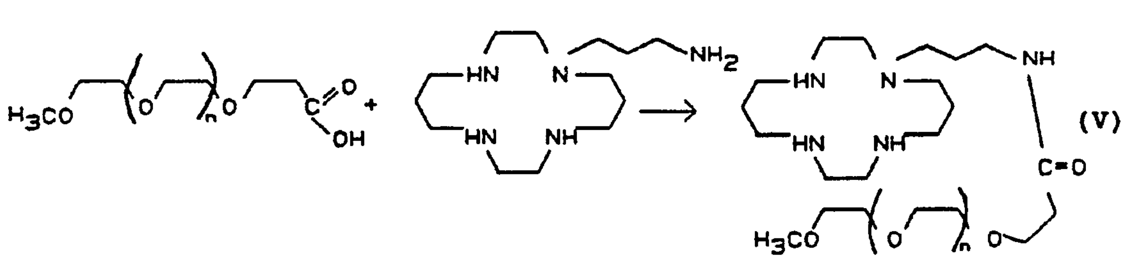

(V)

368 mg des Polymers (MW = 5000), dargestellt nach K. Geckeler, E. Bayer, Polymer Bull. 3 (1980), 347, werden mit 7 mg $N(C_2H_5)_3$, 17,6 mg Cyclamderivat aus Beispiel 1, 15,2 mg Dicyclohexylcarbodiimid und 8,4 mg 1-Hydroxybenzotriazol in 5 ml Ethylacetat, 5 ml N,N-Dimethylformamid und 10 Tropfen Wasser gelöst. Nach 16 h Rühren werden die Lösungsmittel im Vakuum entfernt und dann wird der Rückstand mehrfach in wäßriger Lösung ultrafiltriert. Aus dem Konzentrat können 345 mg Produkt (V) (90%) isoliert werden. (kein definierter Fp., da Polymer).

b) Markierung von (V)

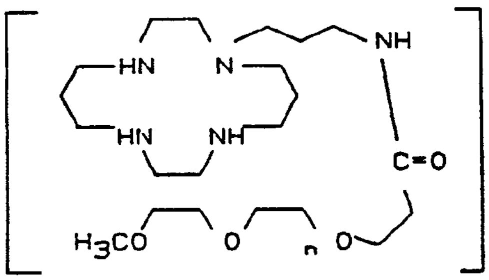

mit $^{99m}Tc$

8 mg des Polymerkonjugats aus Beispiel 3a) werden in 0,5 ml 0,9% NaCl gelöst, der pH mit 0,1 N NaOH auf 11 eingestellt, 1 ml der in der deutschen Offenlegungsschrift DE-A 3728599 (= EP-A 0271806 und US Ser.No. 130183) in Beispiel 7 beschriebenen PTP-Sn-Komponente dazugegeben (gelöst in 0,9 N NaCl), dann werden 0,3 ml $^{99m}TcO_4^-$- Lösung (ca. 59 MBq $^{99m}Tc$) dazugegeben. Nach 5 Minuten bei Raumtemperatur liegt der pH bei ca. 7. Dünnschichtchromatographie im Vergleich zu unsubstituiertem Cyclam, freiem Pertechnetat, dem Ausgangspolymer sowie der PTP-Komponente, jeweils unter gleichen Bedingungen 5 Minuten nach Pertechnetatzugabe chromatographiert, ergab :

Ca. 10% des eingesetzten $^{99m}Tc$ liegen an das Polymerkonjugat gebunden vor. Die diesem Ergebnis zugrundeliegenden Chromatographiedaten sind in Tabelle 1 zusammengefaßt.

| | |
|---|---|
| (Chromatographiesysteme | ITLC SG/Methylethylketon |
| | ITLC SG/2 m $Na_2CO_3$ |
| | Cellulose auf Al/10% $NH_4OOCCH_3$ : $CH_3OH$ 1 : 1 |
| | Whatman 1 Papier/0,9% NaCl |
| | ITLC SG/0,5 M $NH_4OOCCH_3$) |

**Tabelle 1**

| Chromato- graphie- Substanz system | ITLC SG Methyl- ethylketon | ITLC SG 2M $Na_2CO_3$ | Cellulose/Al 10% $NH_4OAc$:MeOH 1:1 | Whatman/ 0,9% NaCl | ITLC SG 0,5M $NH_4OAc$ |
|---|---|---|---|---|---|
| $^{99m}Tc$-PEG | Rf=0 43,2%<br>Rf=1 56,8%<br><br>Rf=0 | Rf=0,6-1 100%<br><br><br>Rf=0 | Rf=0,1 97,1%<br>Rf=0,7 2,9%<br><br>Rf=1 Rf=1 | Rf=0,1 98,3%<br>Rf=0,8 1,4%<br><br>Rf=1 | Rf=0,1 11,4%<br>Rf=0,2 18,1%<br>Rf=1 70,5%<br>Rf=0-0,3 |
| $^{99m}Tc$-PEG -COOH | Rf=0 67,2%<br>Rf= 32,8%<br><br>Rf=0 | Rf=0,6-1 100%<br><br><br>Rf=0 | Rf=0,1 98,0%<br>Rf=0,7 2,0%<br><br>Rf=1 | Rf=0,1 96,8%<br>Rf=0,8 3,2%<br><br>Rf=1 | Rf=0,1 23,7%<br>Rf=0,2 37,6%<br>Rf=1 48,7%<br>Rf=0-0,2 |
| $^{99m}Tc$-PEG $-NH_2$ Propyl- cyclan | Rf=0 78,6%<br>Rf=1 21,4%<br><br>Rf=0 | Rf=0 9,5 %<br>Rf=0,2-0,9 90,5%<br><br>Rf=0 | Rf=0,1 45,6%<br>Rf=0,7-0,9 54,4%<br><br>Rf=0,7-1 | Rf=0,1 40,2%<br>Rf=0,9 59,8%<br><br>Rf=0,8-1 | Rf=0,1 35,1%<br>Rf=0,2 37,6%<br>Rf=1 27,3%<br>Rf=0-0,1 |
| $^{99m}Tc$- Cyclam | Rf=0,1-0,2 100%<br><br>Rf=0-0,2 | Rf=0 1,5%<br>Rf=0,7-6 98,5%<br>Rf=1 | Rf= 0,9 100%<br><br>Rf=0,9 | Rf=0,1 1,2%<br>Rf=0,4 98,8%<br>nicht anfärbbar | Rf=0-0,3 15,6%<br>Rf=0,3-0,9 84,4%<br>Rf=0,3 Rf=0,3 |
| $^{99m}Tc$- PTP | Rf=0 98,9%<br>Rf=1 1,1% | Rf=0 4,6%<br>Rf=1 95,4% | Rf=0 100% | Rf=0,1 100% | Rf=0,1 29,2%<br>Rf=0,1-0,8 41,3%<br>Rf=0,8-1 10,0% |
| $^{99m}TcO_4^-$ | Rf=0 2,9%<br>Rf=1 97,1% | Rf=1 100% | Rf=0,7 100% | Rf=1 100% | Rf=1 100% |

**Beispiel 5 :**

Darstellung von 2-Hydroxymethyl-1,4,8,11-tetraazacyclotetradecantetrahydrobromid

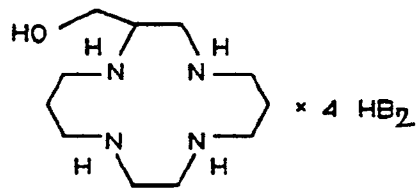

a) und b) Die Sysnthese der Ausgangsverbindung NaTsN-$(CH_2)_3$-NTs-$(CH_2)_2$-NTs-$(CH_2)_3$-NTsNA wird analog Beispiel 2a) und b) durchgeführt, nur das N,N'-(Diaminoethyl)-1,3-propandiamin durch N,N'-(Diaminopropyl)-1,2-ethylendiamin ersetzt wird.

c) Synthese von 1-Phenoxymethyl-1,2-ethandiol

Der Phenylglycidylether wird nach der Literaturvorschrift von D.M. Dishong, C.J. Diamond, M.I. Cinoman, G.W. Gokel, J.Am. Chem. Soc. 105 (1983), 586-593 dargestellt.

d) Darstellung von 1-Phenoxymethyl-1,2-ethandiol-ditosylat

5 g des Ausgangsdiols (c) werden in 25 ml Pyridin vorgelegt und 10,5 g p-Toluylsulfonsäurechlorid dazugegeben (leicht exotherme Reaktion !). Nach ca. 30 Minuten tritt Niederschlag auf. Das Gemisch wird weitere 5 h bei Raumtemperatur gerührt, 20 h stehen gelassen und dann das Pyridin im Vakuum entfernt. Der Rückstand wird in 2 N HCl aufgenommen, 2 × mit Diethylether extrahiert, die etherischen Phasen mit $Na_2SO_4$ getrocknet, filtriert und das Produkt durch Einengen gewonnen. Ausbeute 11,2 g Feststoff (83% d.Th.). Identifikation durch $^1H$-NMR ($CDCl_3$) und Dünnschichtchromatographie (Kieselgel, $CH_2Cl_2$, Rf = 0,6).

e) Cyclisierung zu 2-Phenoxymethyl-1,4,8,11-Tetratosyl-1,4,8,11-tetraazacyclotetradecan

8,53 g des 1,12-Dinatriumsalzes des 1,5,8,12-Tetratosyl-1,5,8,12-Tetraazadodecans (wie unter b) beschrieben) werden in ca. 40 ml N,N-Dimethylformamid (DMF) vorgelegt, auf 100°C erhitzt und dann eine Lösung von 4,75 g 1-Phenoxymethyl-1,2-ethandiol-ditosylat in 40 ml DMF dazugetropft. Weitere 6 h wird bei 100°C gerührt, gefolgt von 20 h Rühren bei 20°C. Dann tropft man ca. 200 ml $H_2O$ zu, rührt eine Stunde lang stark (klebriger Niederschlag), gibt festes NaCl zum Aussalzen zu und filtriert den weißen Niederschlag ab. Der Filterkuchen wird mehrmals mit Wasser gewaschen, dann vakuumgetrocknet. Aus der Mutterlösung kann durch weitere Wasserzugabe etwas mehr Produkt isoliert werden. Ausbeute 4,7 g weißer Feststoff, entspricht 50 % d.Th.. Charakterisierung durch Dünnschichtchromatographie (Kieselgel, Ethylacetat, Rf = 0,8) und $^1H$-NMR ($CDCl_3$).

f) Detosylierung von 2-Phenoxymethyl-1,4,8,11-tetratosyl-1,4,8,11-tetraazacyclotetradecan zu 2-Hydroxymethyl-1,4,8,11-tetraazacyclotetradecan-tetrahydrobromid. Die tetratosylierte Ausgangsverbindung (10,8 g) wird in 80 ml 48% HBr + 80 ml Eisessig 3 Tage am Rückfluß gekocht, 1 Tag bei Raumtemperatur stehengelassen, dann in $CH_2Cl_2/H_2O$ aufgenommen, die org. Phase 1 mal mit $H_2O$ gewaschen, dann die wäßrige Lösung 5 mal mit je 100 ml $CH_2Cl_2$ extrahiert (bis das $CH_2Cl_2$ farblos ist), die wäßr. Phases eingeengt. Der Rückstand (vakuumgetrocknet) wird 13 mal mit Portionen von insgesamt 800 ml $CH_2Cl_2$ im Ultraschallbad trituiert, bis ein weißgelblicher pulvriger Rückstand bleibt. Vakuumtrocknung ergibt ein Produkt, das 2-Hydroxymethyl-1,4,8,11-tetraazacyclotetradecan-tetrahydrobromid mit einer Tosylgruppe entspricht (NMR). Dieses wird in 100 ml mit HBr-Gas gesättigten Eisessig gegeben und 4 Tage rückflußgekocht. Das Rohprodukt fällt als graugelblicher Feststoff aus, der über einen stark basischen Anionenaustauscher gegeben wird (Eluierung mit $H_2O$), um Essigsäure zu entfernen. Ein Massenspektrum zeigt $M^+$ = 230 sowie Bruchstücke bei m/e = 200 (Cyclam) mit m/e = 174 (offenkettiges Ausgangsmaterial). C,H,N-Analyse : 21,8% C, 5,3%, H, 9,9% N, 58,7% Br, berechnet für 2-Hydroxymethyl-1,4,8,11-tetraazacyclotetradecan-tetrahydrobromid : 23,8% C, 5,4% H, 10,1% N, 57,7% Br. Auch das $^1H$-NMR zeigt 2-Hydroxymethyl-1,4,8,11-tetraazacyclotetradecantetrahydrobromid, wobei sich 2-Hydroxymethyl-1,4,8,11-tetraazacyclotetradecantetrahydrobromid von der offenkettigen Ausgangsverbindung (in $D_2O$) hauptsächlich durch ein verändertes Integralverhältnis unterscheidet. Eine nochmalige Reinigung des Produktes (900 mg Ausbeute = 34%) kann durch präparative HPLC (RP8-Säule, Methanol/$H_2O$/HCl/ph 2,7 mit Mischungsgradient, Elutionszeit 8-10 Minuten) erfolgen.

**Beispiel 6 :**

Markierung von

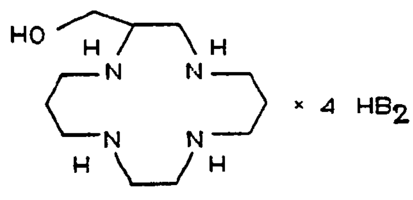

mit $^{99m}Tc$

1 mg 2-Hydroxymethyl-1,4,8,11-tetraazacyclotetradecantetrahydrobromid wird in 1 ml 0,9% NaCl gelöst. 0,1 ml einer Lsg. von 1 mg $SnCl_2 \times 5\ H_2O$ in 1 ml 0,1 N HCl werden zugegeben und der pH-Wert mit 0,1 ml 0,1 N NaOH auf pH 5-6 eingestellt. Nach Zugabe von 0,15 ml $^{99m}Tc$-Eluat wird die Aktivität der Lösung bestimmt. Sie bträgt 79,12 MBq/ml. 30 Minuten später wird ITLC-SG (2 × 10 cm-Streifen)—Chromatographie durchgeführt. Zwei Proben werden gleichzeitig chromatographiert, eine in Methylethylketon (freies $^{99m}TcO_4$-läuft mit der Front), eine in 2 M $Na_2CO_3$ (kolloidales $^{99m}TcO_4$- bleibt am Start sitzen). In Methylethylketon erscheint die gesamte Radioaktivität bei Rf = 0,43, in $Na_2CO_3$ erscheinen ca. 90% der Radioaktivität bei Rf = 1,0, der Rest schmiert zwischen Rf = 0,4 und 1,0. Dies bedeutet, daß $\geqq$ 90% des Technetiums komplexiert sind.

## Patentansprüche

1. Verfahren zur Markierung von Substanzen mit Technetium- bzw. Rheniumisotopen, dadurch gekennzeichnet, daß

a) die zu markierende Substanz mit einem N-substituierten oder C-substituierten macrocyclischen Aminderivat der Formel I oder II

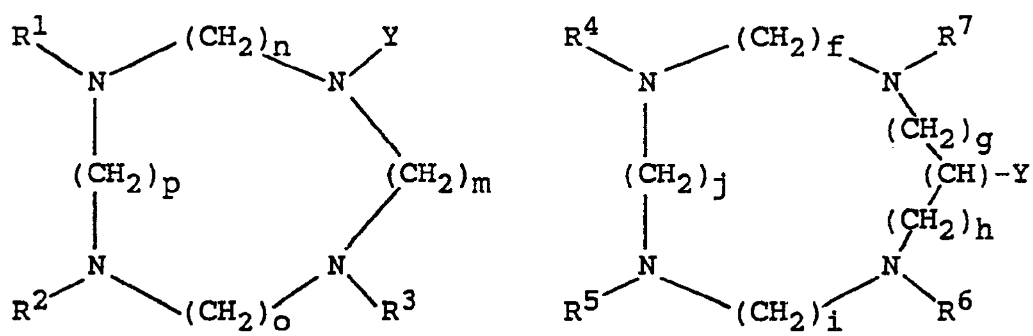

worin
$R^1$, $R^2$, $R^3$, $R^4$, $R^5$, $R^6$ und $R^7$ Wasserstoff bedeuten und
m, n, o, p, f, h, i, j gleich oder verschieden sind und 1, 2, 3 oder 4 und g 0, 1, 2, 3 oder 4 bedeuten und
Y eine Gruppe der Formel —X-$NH_2$, —X-NCS, —X-COOH, —X-OH, —X-$N_2^+$ oder —X-COCl oder eine Gruppe der Formel —X-Z bedeutet, wobei Z, Fluor, Chlor, Brom oder Jod bedeutet und X eine Alkylengruppe mit 1 bis 40 C-Atomen bedeutet oder X eine ortho-, meta- oder para-Phenylen oder eine ortho-, meta- oder para-Phenylmethylgruppe bedeutet,
umgesetzt wird und die so erhaltene Verbindung mit Technetium-99m oder Rhenium-186 oder -188 markiert wird, in dem man sie mit Pertechnetat-99m oder Perrhenat-186 oder -188 und einem Reduktionsmittel für Pertechnetat-99m oder Perrhenat-186 oder -188 versetzt oder daß man

b) zunächst den Technetium-99m- und/oder Rhenium-186 oder -188-Komplex durch Umsetzung einer Verbindung der Formel I und/oder II mit Pertechnetat-99m und/oder Perrhenat-186 oder -188 und einem Reduktionsmittel für Pertechnetat-99m und/oder Perrhenat-186 oder -188 herstellt und anschließend diesen Technetium-99m- und/oder Rhenium-186 oder -188-Komplex mit der zu markierenden Substanz umsetzt.

2. Verfahren nach Anspruch 1, gekennzeichnet durch makrocyclische Aminderivate der Formel I oder II gemäß Anspruch 1, worin

$R^1$, $R^2$, $R^3$, $R^4$, $R^5$, $R^6$ und $R^7$ Wasserstoff bedeuten und

m, n, o, p, f, i und j gleich oder verschieden sind und 2 oder 3 und g 0, 1 oder 2 und h 1 oder 2 bedeutet und

Y eine Gruppe der Formel $—X\text{-}NH_2—$, —X-NCS, —X-COOH, —X-OH, $—X\text{-}N_2^+$ oder —X-COCl oder eine Gruppe der Formel —X-Z bedeutet, wobei Z Fluor, Chlor, Brom oder Jod bedeutet und X eine Alkylengruppe mit 1 bis 20 C-Atomen bedeutet.

3. Verfahren nach Anspruch 1, gekennzeichnet durch makrocyclische Aminderivate der Formel I oder II, gemäß Anspruch 1, worin

$R^1$, $R^2$, $R^3$, $R^4$, $R^5$, $R^6$ und $R^7$ Wasserstoff bedeuten und

m, p und j 3 und n, o, f und i 2 und g und h 1 bedeuten und

Y eine Gruppe der Formel $—X\text{-}NH_2$ oder —X-OH bedeutet, wobei X eine Alkylengruppe mit 1 bis 15 C-Atomen bedeutet.

4. Verfahren nach Anspruch 1, dadurch gekennzeichnet, daß die zu markierende Substanz eine reaktive Gruppe enthält, die mit der reaktiven Gruppe, die sich an der exocyclischen Seitenkette des macrocylischen Amins befindet, unter Ausbildung einer chemischen Bindung reagiert.

5. Verfahren nach Anspruch 4, dadurch gekennzeichnet, daß die reaktive Gruppe der zu markierenden Substanz eine $—NH_2—$, —COOH—, —COCl—, —OH— oder $—NH\text{-}NH_2$-Gruppe ist.

6. Verfahren nach Anspruch 4, dadurch gekennzeichnet, daß die zu markierende Substanz ein Antikörper, ein Antikörperfragment, ein monoklonaler Antikörper, ein monoklonales Antikörperfragment ($F(ab')_2$- oder F(ab')-Fragment), ein Protein, ein Enzym, ein Zucker, eine Fettsäure, ein Hormon oder ein Polymer ist.

7. Verfahren nach Anspruch 6, dadurch gekennzeichnet, daß das Polymer mindestens eine Seitenkette der Formel $—B\text{-}NH_2$, —B-COOH, $—B\text{-}NH\text{-}NH_2$, —B-COCl oder —B-OH enthält, wobei B einen o-, m- oder p-Arylrest oder eine Alkylenkette mit 1 bis 40 C-Atomen darstellt, die im Falle einer $C_2$-$C_{40}$-Alkylenkette auch durch eine —NH—, —NH-CO—, —CO-NH—, —CO-O— oder —O-CO-Einheit unterbrochen sein kann und wobei das Polymer ein Molekulargewicht von 1000 bis 200.000 (Dalton) besitzt.

8. Testkit zum Nachweis von Tumoren, bestehend aus zwei getrennten, lyophilisierten Komponenten, von denen eine einen Antikörper, der gegen Tumor-assoziierte Antigene gerichtet ist, oder dessen $F(ab')_2$-Fragment im Gemisch mit einem Puffer, wobei der Antikörper oder dessen Fragment mit einem makrocyclischen Amin nach Anspruch 1 verknüpft ist, und die andere ein Reduktionsmittel enthält, das zur Reduktion und Bindung des Technetiums an der Antikörperkomponente benötigt wird.

9. Testkit nach Anspruch 8, dadurch gekennzeichnet, daß das Reduktionsmittel ein Zinn-II-Salz ist.

10. Testkit nach Anspruch 9, dadurch gekennzeichnet, daß das Zinn-II-Salz der Methandiphosphonsäure, Aminomethandiphosphonsäure, 3,3-Diphosphonopropionsäure, 3,3-Diphosphono-1,2-propandicarbonsäure oder der Propan-1,1,3,3-tetraphosphonsäure eingesetzt wird.

11. Diagnostikum, dadurch hergestellt, daß man eine lyophilisierte organspezifische Substanz, die chemisch an ein makrocyclisches Amin nach Anspruch 1 gebunden ist, in einer Technetium-99m-Pertechnetat-Lösung auflöst und dann die Reduktion und Bindung des Technetiums an das macrocyclische Amin durch Zugabe eines Reduktionsmittels bewirkt.

12. Diagnostikum, dadurch hergestellt, daß man die lyophilisierte organspezifische Substanz, die chemisch an ein makrocyclisches Amin nach Anspruch 1 gebunden ist, zuerst in der Lösung des Reduktionsmittels auflöst, und anschließend durch Zugabe von Technetium-99m-Pertechnetat-Lösung das macrocyclische Amin und damit die organspezifische Substanz mit Technetium-99m markiert.

13. Therapeutikum, enthaltend eine organspezifische Substanz, welche chemisch an ein mit Rhenium-186 oder Rhenium-188 markiertes macrocyclisches Amin nach Anspruch 1 gekoppelt ist.

## Claims

1. A method for labeling substances with technetium or rhenium isotopes, which comprises

a) the substance which is to be labeled being reacted with an N-substituted or C-substituted macrocyclic amine derivative of the formula I or II

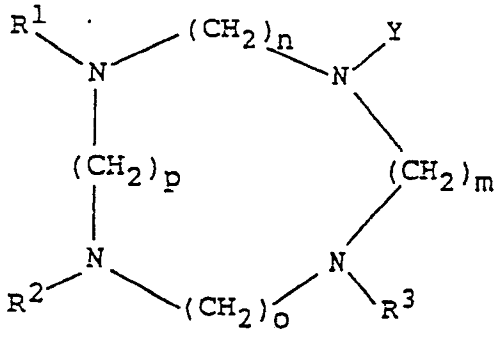

I

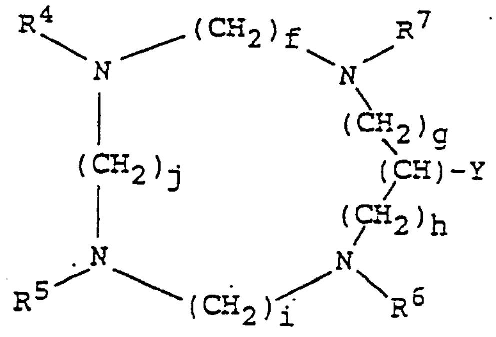

II

in which

$R^1$, $R^2$, $R^3$, $R^4$, $R^5$, $R^6$ and $R^7$ denote hydrogen, and

m, n, o, p, f, h, i and j are identical or different and denote 1, 2, 3 or 4 and g denotes 0, 1, 2, 3 or 4, and

Y denotes a group of the formula —X-$NH_2$, —X-NCS, —X-COOH, —X-OH, —X-$N_2^+$ or —X-COCl or a group of the formula —X-Z, where Z denotes fluorine, chlorine, bromine or iodine, and X denotes an alkylene group having 1 to 40 carbon atoms, or X denotes an ortho-, meta- or para-phenylene or an ortho-, meta- or para-phenylenemethyl group,

and the resulting compound being labeled with technetium-99m or rhenium-186 or -188 by adding to it pertechnetate-99m or perrhenate-186 or -188 and a reducing agent for pertechnetate-99m or perrhenate-186 or -188, or comprises

b) initially preparing the technetium-99m and/or rhenium-186 or -188 complex by reaction of a compound of the formula I and/or II with pertechnetate-99m and/or perrhenate-186 or -188 and a reducing agent for pertechnetate-99m and/or perrhenate-186 or -188, and subsequently reacting this technetium-99m and/or rhenium-186 or -188 complex with the substance which is to be labeled.

2. The method as claimed in claim 1, wherein are used macrocyclic amine derivatives of the formula I or II as claimed in claim 1, in which

$R^1$, $R^2$, $R^3$, $R^4$, $R^5$, $R^6$ and $R^7$ denote hydrogen, and

m, n, o, p, f, i and j are identical or different and denote 2 or 3, and g denotes 0, 1 or 2, and h denotes 1 or 2, and

Y denotes a group of the formula —X-$NH_2$, —X-NCS, —X-COOH, —X-OH, —X-$N_2^+$ or —X-COCl or a group of the formula —X-Z, where Z denotes fluorine, chlorine, bromine or iodine, and X denotes an alkylene group having 1 to 20 carbon atoms.

3. The method as claimed in claim 1, wherein are used macrocyclic amine derivatives of the formula I or II as claimed in claim 1, in which

$R^1$, $R^2$, $R^3$, $R^4$, $R^5$, $R^6$ and $R^7$ denote hydrogen, and

m, p and j denote 3, and n, o, f and i denote 2, and g and h denote 1, and

Y denotes a group of the formula —X-$NH_2$ or —X-OH, where X denotes an alkylene group having 1 to 15 carbon atoms.

4. The method as claimed in claim 1, wherein the substance which is to be labeled contains a reactive group which reacts with the reactive group which is located in the exocyclic side-chain of the macrocyclic amine, with the formation of a chemical bond.

5. The method as claimed in claim 4, wherein the reactive group on the substance which is to be labeled is an —$NH_2$, —COOH, —COCl, —OH or —NH-$NH_2$ group.

6. The method as claimed in claim 4, wherein the substance which is to be labeled is an antibody, an antibody fragment, a monoclonal antibody, a monoclonal antibody fragment ($F(ab')_2$ or F(ab') fragment), a protein, an enzyme, a sugar, a fatty acid, a hormone or a polymer.

7. The method as claimed in claim 6, wherein the polymer contains at least one side-chain of the formula —B-$NH_2$, —B-COOH, —B-NH-$NH_2$, —B-COCl or —B-OH, where B represents an o-, m- or p-arylene radical or an alkylene chain having 1 to 40 carbon atoms which, in the case of a $C_2$-$C_{40}$-alkylene chain, can also be interrupted by an —NH, —NH-CO—, —CO-NH—, —CO-O— or —O-CO— unit, and where the polymer has a molecular weight of 1000 to 200,000 (Dalton).

8. A test kit for detecting tumors, composed of two separate lyophilized components, one of which contains an antibody which is directed against tumor-associated antigens, or the $F(ab')_2$ fragment thereof, mixed with

a buffer, with the antibody or the fragment thereof being linked to a macrocyclic amine as claimed in claim 1, and the other contains a reducing agent which is required for the reduction and binding of the technetium to the antibody component.

9. A test kit as claimed in claim 8, wherein the reducing agent is a tin(II) salt.

10. A test kit as claimed in claim 9, wherein the tin(II) salt of methanediphosphonic acid, aminomethanedisphosphonic acid, 3,3-diphosphonopropionic acid, 3,3-diphosphono-1,2-propanedicarboxylic acid or propane-1,1,3,3-tetraphosphonic acid is employed.

11. A diagnostic aid which is prepared by dissolving a lyophilized organ-specific substance which is chemically bonded to a macrocyclic amine as claimed in claim 1 in a technetium-99m-pertechnetate solution, and then bringing about the reduction and binding of the technetium to the macrocyclic amine by addition of a reducing agent.

12. A diagnostic aid which is prepared by first dissolving the lyophilized organ-specific substance which is chemically bonded to a macrocyclic amine as claimed in claim 1 in the solution of the reducing agent, and subsequently labeling the macrocyclic amine, and thus the organ-specific substance, with technetium-99m by addition of technetium-99m-pertechnetate solution.

13. A therapeutic agent containing an organ-specific substance which is chemically coupled to a macrocyclic amine as claimed in claim 1 labeled with rhenium-186 or rhenium-188.

## Revendications

1. Procédé pour marquer des substances avec des isotopes de technétium ou de rhénium, caractérisé en ce que

a) on fait réagir la substance à marquer avec un dérivé aminé macrocyclique N- ou C-substitué de formule I ou II

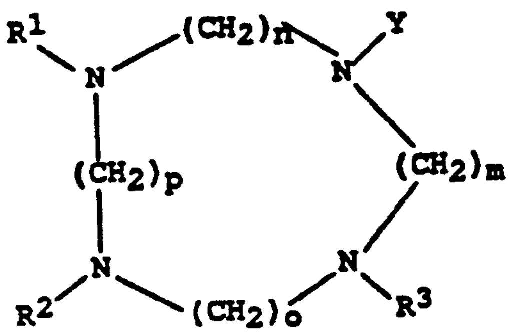

I

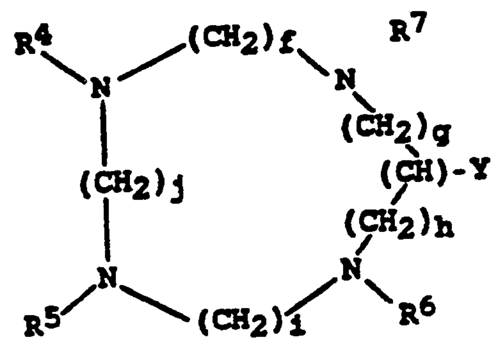

II

dans lesquelles

$R^1$, $R^2$, $R^3$, $R^4$, $R^5$, $R^6$ et $R^7$ représentent un atome d'hydrogène, et

m, n, o, p, f, h, i, j sont identiques ou différents et sont égaux à 1, 2, 3 ou 4 et g est égal à 0, 1, 2, 3 ou 4, et

Y représente un groupe de formule —X-$NH_2$, —X-NCS, —X-COOH, —X-OH, —X-$N_2^+$ ou —X-COCl ou un groupe de formule—X-Z, Z représentant un atome de fluor, de chlore, de brome ou d'iode et X représentant un groupe alkylène avant 1 à 40 atones de carbone ou X représentant un groupe ortho-, méta- ou para-phénylène ou un groupe ortho-, métaou para-phénylèneméthyle,

et on marque le composé ainsi obtenu avec le technétiun-99m ou le rhéniun-186 ou -188, dans lequel on les fait réagir avec le pertechnétate-99m ou le perrhénate-186 ou -188 et un agent réducteur du pertechnétate-99m ou du perrhénate-186 ou -188, ou que l'on

b) prépare d'abord le complexe de technétium-99m et/ou de rhénium-186 ou -188 par réaction d'un composé de formule I et/ou II avec le pertechnétate-99m et/ou le perrhénate-186 ou -188 et un agent réducteur du pertechnétate-99m et/ou du perrhénate-186 ou -188 et on fait réagir ensuite ce complexe de technétium-99m et/ou de rhénium-186 ou -188 avec la substance à marquer.

2. Procédé selon la revendication 1, caractérisé par des dérivés aminés macrocycligues de formule I ou II conformément à la revendication 1, dans lesquelles

$R^1$, $R^2$, $R^3$, $R^4$, $R^5$, $R^6$ et $R^7$ représentent un atome d'hydrogène, et

m, n, o, p, f, i et j sont identiques ou différents et sont égaux à 2 ou à 3 et g est égal à 0, 1 ou 2 et h est égal à 1 ou à 2 et,

Y représente un groupe de formule —X-$NH_2$, —X-NCS, —X-COOH, —X-OH, —X-$N_2^+$ ou —X-COCl ou un groupe de fomule —X-Z, Z représentant un atome de fluor, de chlore, de brome ou d'iode et X représentant un groupe alkylène ayant 1 à 20 atomes de carbone.

3. Procédé selon la revendication 1. caractérisé par des dérivés aminés macrocycliques de formule I ou II conformément à la revendication 1, dans lesquelles

$R^1$, $R^2$, $R^3$, $R^4$, $R^5$, $R^6$ et $R^7$ représentent un atome d'hydrogène, et

m, p et j sont égaux à 3 et n, o, f et i sont égaux à 2 et g et h sont égaux à 1, et

Y représente un groupe de formule —X-$NH_2$ ou —X-OH, X représentant un groupe alkylène ayant 1 à 15 atomes de carbone.

4. Procédé selon la revendication 1, caractérisé en ce que la substance à marquer contient un groupe réactif qui réagit avec le groupe réactif situé sur la chaîne latérale exocyclique de l'amine macrocyclique, par formation d'une liaison chimique.

5. Procédé selon la revendication 4, caractérisé en ce quele groupe réactif de la substance à marquer est un groupe —$NH_2$—, —COOH—, —COCl—, —OH— ou —NH-$NH_2$—.

6. Procédé selon la revendication 4, caractérisé en ce quela substance à marquer est un anticorps, un fragment d'anticorps, un anticorps monoclonal, un fragment d'anticorps monoclonal (fragment F(ab')2 ou F(ab')), une protéine, une enzyme, un sucre, un acide gras, une hormone ou un polymère.

7. Procédé selon la revendication 6, caractérisé en ce que le polymère contient au moins une chaîne latérale de formule —B-$NH_2$, —B-COOH, —B-NH-$NH_2$, —B-COCl ou —B-OH, B représentant un radical o-, m- ou p-aryle ou une chaîne alkylène ayant 1 à 40 atomes de carbone, gui peut également être interrompue, dans le cas d'une chaîne alkylène en $C_2$-$C_{40}$, par un chaînon —NH—, —NH-CO—, —CO-NH—, —CO-O— ou —O-CO—, et le polymère ayant une nasse moléculaire comprise entre 1000 et 200000 (Dalton).

8. Coffret d'analyse pour le dépistage de tumeurs,constitué de deux composants distincts lyophilisés, dont l'un contient un anticorps qui est dirigé contre des antigènes associés à la tumeur, ou un fragment $F(ab')_2$ de celui-ci mélangé avec un tampon ; l'anticorps ou son fragment étant lié à une amine macrocyclique selon la revendication 1, et l'autre contient un agent réducteur, nécessaire à la réduction du technétium et à sa liaison au composant anticorps.

9. Coffret d'analyse selon la revendication 8, caractérisé en ce que l'agent réducteur est un sel d'étain II.

10. Coffret d'analyse selon la revendication 9, caractérisé en ce que l'on utilise le sel d'étain II de l'acide méthanediphosphonique, de l'acide aminométhanediphosphonique, de l'acide 3,3-diphosphonopropionique, de l'acide 3,3-diphosphono-1,2-propanedicarboxylique ou de l'acide propane-1,1,3,3-tétraphosphonique.

11. Agent de diagnostic, préparé par dissolution d'une substance lyophilisée, spécifique d'un organe, qui est chimiquement liée à une amine macrocyclique selon la revendication 1, dans une solution de pertechnétate autechnétium-99m et ensuite par la réalisation de la réduction du technétium et sa liaison à l'amine macrocyclique par addition d'un agent réducteur.

12. Agent de diagnostic, préparé par dissolution d'abord de la substance lyophilisée, spécifique d'un organe, qui est chimiquement liée à une amine macrocyclique selon la revendication 1, dans la solution de l'agent réducteur et ensuite marquage au technétium-99m, par addition d'une solution de pertechnétate au technétium-99m, de l'amine macrocyclique et ce faisant, de la substance spécifique d'un organe.

13. Agent thérapeutique, comprenant une substance spécifique d'un organe, laquelle est chimiquement couplée à une amine macrocyclique, marquée au rhénium-186 ou -188 selon la revendication 1.